# EUROPEAN PATENT APPLICATION

(11) **EP 4 704 109 A1**
(43) Date of publication of application: **04.03.2026**
(21) Application number: 25165171.7
(22) Date of filing: 20.03.2025
(51) Int. Cl.: G16H 40/63, G16H 30/00, A61B 6/02, A61B 6/46, A61B 6/50, G06F 3/0482, G06F 3/0484, G06T 3/40

(54) **DISPLAYING GROUPED MEDICAL IMAGES FOR REVIEW**

(30) Priority: 30.08.2024 US 202418820496
(71) Applicant: FUJIFILM Healthcare Americas Corporation, Lexington, MA 02421 (US)
(72) Inventor: MULLIGAN, Jasmine, Seattle, 98115 (US); PRABHAKARAN, Prajeesh, Morrisville, 27560 (US); O'KEEFE, Myles, Cary, 27513 (US)
(74) Representative: Dehns

(57) **Abstract**

Systems and methods are described that implement displaying grouped medical images for review. Current medical image management systems generally use thumbnail image grouping of images that have the same position and side. These groups of thumbnail images can be large, making it difficult for a clinician (e.g., a radiologist) to quickly find a particular view they are interested in reviewing. The disclosed systems and methods provide for user-selectable grouping and ungrouping of thumbnail images in a graphical user interface (300; 400), which makes it easier for a clinician to quickly find a particular view that they are interested in and enables a more-efficient review of an imaging study.

## Description

To diagnose patients, medical professionals often order imaging studies that include medical image data. These imaging studies are then read by a clinician (e.g., a radiologist) to create an interpretation report. For the reading, the medical images for the study are displayed for the clinician's reading in a graphical user interface of a display device.

In some cases, the medical images are grouped together into a set, with each set represented by a thumbnail image, which is provided to enable the clinician to navigate the contents of the study. The set-grouped medical images include images that have the same imaging position (e.g., craniocaudal (CC), mediolateral oblique (MLO)) and side (e.g., left, right).

When the imaging study is generated through a mammogram, the set-grouped mammographic images may include one or more x-ray projection images (aka "2D images"), supplementary mammographic views, images of a breast tomosynthesis series, "synthetic" images (e.g., generated from a tomosynthesis scan), technical repeat images, and/or computer-aided diagnosis (CAD) generated images. In a mammographic image review, the clinician may read the mammographic images by comparing right and left breast images to one another (e.g., comparing the right craniocaudal (RCC) view to the left craniocaudal (LCC) view) and/or by comparing different direction images to one another (e.g., comparing the RCC view to the RMLO view). Additionally, the grouping can make it difficult for the clinician to tell, just by looking at the thumbnail image, that a technical repeat has occurred. As a result, a clinician may not be prompted to view a particular medical image they would have found useful to their diagnosis.

To handle the display of sets that include multiple images, it is common to configure the GUI to display any additional images of a set as additional pages within the viewport. For example, if a set includes more than one image, a button(s) may be presented to enable the clinician to scroll (e.g., page up, page down) between images of the set in the viewport. For large sets of images, the clinician may need to page up/down through multiple images looking for a particular view, which results in the clinician taking longer to complete their read. As a result of the set grouping of images that have the same position and side, it can be difficult for a clinician to quickly find, via the thumbnail images, a particular view that they are interested in reviewing.

Systems and methods for displaying grouped medical images for review are disclosed. Current medical image management systems generally use thumbnail image grouping of images that have the same position and side. These groups of thumbnail images can be large, making it difficult for a clinician (e.g., a radiologist) to quickly find a particular view they are interested in reviewing. The disclosed systems and methods provide for user-selectable grouping and ungrouping of thumbnail images in a graphical user interface, which makes it easier for a clinician to quickly find a particular view that they are interested in and enables a more efficient review of an imaging study.

According to a first aspect, the present invention provides a method including: receiving an image data set from a data source, the image data set including projection X-ray mammographic images for a healthcare study; determining an ungrouped thumbnail image for each projection X-ray mammographic image of the image data set, the ungrouped thumbnail images for display in a graphical user interface of a display device; grouping images of the image data set into a plurality of sets; determining a set-grouped thumbnail image for each set of grouped images, the set-grouped thumbnail images for display in the graphical user interface; generating a graphical user interface that includes displaying at least one of the set-grouped thumbnail images or the ungrouped thumbnail images in the graphical user interface; receiving user input on a user interface element; and, in response to receiving user input on the user interface element: switching the graphical user interface from displaying set-grouped thumbnail images in the graphical user interface to displaying ungrouped thumbnail images in the graphical user interface; or switching the graphical user interface from displaying ungrouped thumbnail images in the graphical user interface to displaying set-grouped thumbnail images in the graphical user interface.

According to a second aspect, the present invention provides a medical image management system including: a network communication interface to receive healthcare studies; a memory coupled to the network communication interface to store received healthcare studies; a display screen coupled to the memory to display the received healthcare studies; and one or more processors coupled to the network communication interface, the memory, and the display screen and configured to: receive an image data set from a data source, the image data set including projection X-ray mammographic images for a healthcare study; determine an ungrouped thumbnail image for each projection X-ray mammographic image of the image data set, the ungrouped thumbnail images for display in a graphical user interface of a display device; group images of the image data set into a plurality of sets; determine a set-grouped thumbnail image for each set of grouped images, the set-grouped thumbnail images for display in the graphical user interface; generate a graphical user interface that includes displaying at least one of the set-grouped thumbnail images or the ungrouped thumbnail images in the graphical user interface; receive user input on a user interface element; and, in response to receiving user input on the user interface element: switch the graphical user interface from displaying set-grouped thumbnail images in the graphical user interface to displaying ungrouped thumbnail images in the graphical user interface; or switch the graphical user interface from displaying ungrouped thumbnail images in the graphical user interface to displaying set-grouped thumbnail images in the graphical user interface.

According to a third aspect, the present invention provides a non-transitory computer-readable storage media having instructions stored thereupon, which, when executed by a system having at least one processor, a memory, and a display screen therein, cause the system to perform a method including: receiving an image data set from a data source, the image data set including projection X-ray mammographic images for a healthcare study; determining an ungrouped thumbnail image for each projection X-ray mammographic image of the image data set, the ungrouped thumbnail images for display in a graphical user interface of a display device; grouping images of the image data set into a plurality of sets; determining a set-grouped thumbnail image for each set of grouped images, the set-grouped thumbnail images for display in the graphical user interface; generating a graphical user interface that includes displaying at least one of the set-grouped thumbnail images or the ungrouped thumbnail images in the graphical user interface; receiving user input on a user interface element; and, in response to receiving user input on the user interface element: switching the graphical user interface from displaying set-grouped thumbnail images in the graphical user interface to displaying ungrouped thumbnail images in the graphical user interface; or switching the graphical user interface from displaying ungrouped thumbnail images in the graphical user interface to displaying set-grouped thumbnail images in the graphical user interface.

The concepts for displaying grouped medical images for review summarised above are further described below in the Detailed Description and are illustrated in the Drawings.

The appended drawings illustrate examples and are, therefore, example embodiments of the invention, and are not considered to be limiting in scope.
FIG. 1 illustrates an example environment for a medical image management system.
FIG. 2 is a block diagram showing an example of a computing system architecture for displaying grouped medical images for review.
FIG. 3 illustrates an example graphical user interface display that may be used in the system of FIGs. 1 and 2 for displaying grouped medical images for review.
FIG. 4 illustrates an example graphical user interface display that may be used in the system of FIGs. 1 and 2 for displaying grouped medical images for review.
FIG. 5 depicts a method for displaying grouped medical images for review.
FIG. 6 illustrates an example aspect of a logical representation of a medical image management system that displays grouped medical images for review.

Disclosed are systems and methods for displaying grouped medical images for review. Through utilization of the improved systems and methods described herein, clinicians (e.g., radiologists) can quickly find medical image data (e.g., mammographic images) they need to complete their review, thereby reducing the amount of time required for review, which improves the clinician's user experience, enables the clinician to work more efficiently, and improves the timeliness of patient care.

To diagnose patients, medical professionals (e.g., doctors) often order imaging studies (e.g., healthcare studies) for the imaging of a body part (e.g., breast, pelvis, chest) that generate medical image data, which is used by the clinician to create an interpretation report. For example, in a screening mammogram study, an X-ray imaging device is utilized to generate mammographic images of the breast(s). The mammographic images may include one or more projection X-ray images (aka a "2D images") of the breast imaged from an imaging position (e.g., cranialcaudal (CC), mediolateral oblique (MLO)) and a side (e.g., left, right). In some cases, multiple images may be generated in a study for the same imaging position and/or same side.

An imaging study (e.g., a mammogram imaging study) may include images in addition to the projection X-ray images. In a first example, an image processing application may be utilized on an existing image (e.g., a 2D image) to generate computer-generated images that enhance target tissue in the 2D image. In a second example, images of a breast tomosynthesis series may be captured. In a third example, synthetic images (e.g., computer-generated from a tomosynthesis scan) may be captured. In a fourth example, technical repeat images that were taken during a different compression of the breast may be captured. For example, if a technologist operating an imaging device determines that a captured mammographic image is inadequate (e.g., includes a visible skin fold, inadequately visualizes the tissue, motion blur is present), the technologist may capture one or more additional mammographic images to avoid calling a patient back for additional imaging. In a fifth example, supplementary mammographic views (e.g., exaggerated craniocaudal lateral (XCCL), exaggerated craniocaudal medial (XCCM), lateromedial (LM), and the like) may be captured. In a sixth example, mammographic images for a study may be analyzed via computer-aided diagnosis (CAD) (e.g., computer-aided detection (CADe)) and annotated mammographic images (e.g., CAD-generated images) for the imaging study may be generated.

The mammographic images for the study are displayed for the clinician's reading in a graphical user interface (GUI) of a display device (e.g., a display monitor). In one example, the GUI includes a 2x2 grid of four viewports configured in an upper left viewport, a lower left viewport, an upper right viewport, and a lower right viewport. In such an example configuration, the images are stacked horizontally by imaging position (view) and are stacked vertically by same side (laterality). In their review, the clinician may read the mammographic images by comparing right and left breast images to one another (e.g., comparing a RCC view to a LCC view) and/or by comparing different direction images to one another (e.g., comparing the RCC view to a RMLO view).

The mammographic images are grouped together into sets, which may include one or more image series. The set-grouped mammographic images include images that have the same imaging position (e.g., CC, LMO) and side (e.g., left, right). In this way, the set-grouped images may include 2D images, supplementary mammographic views, tomosynthesis series images, synthetic images, technical repeat images, and/or computer-aided diagnosis (CAD) generated images. The images (e.g., views) grouped together may represent different series of images (e.g., a 2D image series, a tomosynthesis image series, a synthetic image series, a technical repeat series). Each set grouping is, on the GUI, represented by a thumbnail image (also referred to herein as a "thumbnail"). The term "thumbnail image" broadly refers to a reduced display size image (e.g., a miniature image, an image that is identical other than scale, a reduced resolution image, a cropped image, a symbol, an icon, a simplified depiction image, a line drawing, and the like).

The thumbnail is provided to enable the clinician to navigate the contents of the study. The set grouping is typically provided in order to reduce the number of reading protocol steps for the read and to display a mixed set of images from different mammographic image series in the viewport(s). However, as a result of the set grouping of images that have the same position and side, it can be difficult for a clinician to quickly find a particular view that they are interested in reviewing via thumbnail image navigation. Additionally, the typical set grouping can make it difficult for the clinician to tell, just by looking at the thumbnails, that a technical repeat has occurred. As a result, a clinician may not be prompted to view a particular mammographic image they would have found useful to their diagnosis.

**In** contrast, consider the disclosed systems and methods for displaying grouped medical images for review, which enable clinicians to quickly find and view images they need to complete their review. For example, rather than stepping back through a reading protocol and using page-up/page-down scrolling to view images of a series, a clinician can ungroup thumbnail images to find a thumbnail image of an image for which the clinician is looking, which can then be viewed in the viewport (e.g., by dragging and dropping the thumbnail into a viewport). In this way, a better overview of the study can be provided to the clinician, thereby reducing the amount of time required for review, improving the clinician's user experience, enabling the clinician to work more efficiently, and improving the timeliness of patient care.

**In** the following description, details are set forth to provide a more thorough explanation of the disclosed systems and methods for displaying grouped medical images for review, which may be implemented by a medical image management system. It will be apparent, however, to one skilled in the art, that the disclosed systems and methods may be practiced without these specific details. **In** other instances, well-known structures and devices are shown in block diagram form, rather than in detail, in order to avoid obscuring the disclosed systems and methods. The subject matter of aspects of the disclosed systems and methods for displaying grouped medical images for review is described with specificity herein to meet statutory requirements. However, the description itself is not intended to limit the scope of this patent. Rather, the inventors have contemplated that the claimed subject matter might also be embodied in other ways, to include different steps or combinations of steps similar to the ones described in this document, in conjunction with other present or future technologies.

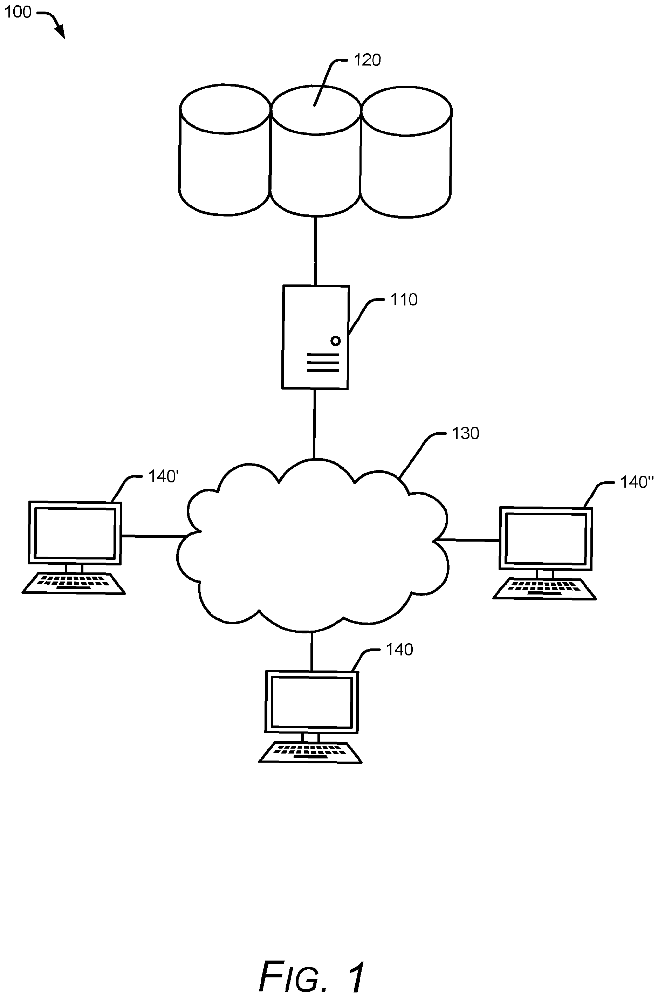

Having briefly described an overview of the disclosed systems and methods for displaying grouped medical images for review, aspects will be discussed with reference to FIGs. 1-6.

Referring to the drawings in general, and initially to FIG. 1 in particular, a medical image management system environment 100, with which aspects of the disclosed systems and methods may be implemented, is illustrated. It will be understood and appreciated by those of ordinary skill in the art that the illustrated environment 100 is merely an example of one suitable computing environment and is not intended to suggest any limitation as to the scope of use or functionality of the disclosed systems and methods. Neither should the environment 100 be interpreted as having any dependency or requirement relating to any single component or combination of components illustrated therein. The disclosed systems and methods may be operational with numerous general-purpose or special-purpose computing system environments or configurations. Examples of well-known computing systems, environments, and/or configurations that may be suitable for use with the disclosed systems and methods include, by way of example only, personal computers, server computers, hand-held devices, laptop devices, multiprocessor systems, microprocessor-based systems, programmable consumer electronics, network personal computers, minicomputers, mainframe computers, distributed computing environments that include any of the above-mentioned systems or devices, and the like.

Aspects of the disclosed systems and methods may be described in the general context of computer-executable instructions (e.g., program modules) configured for execution by a computer. Generally, program modules include, but are not limited to, routines, programs, objects, components, and data structures that perform particular tasks or implement particular abstract data types. The disclosed systems and methods may also be practiced in distributed computing environments where tasks are performed by remote processing devices that are linked through a communications network. In a distributed computing environment, program modules may be located in association with local and/or remote computer storage media including, by way of example only, memory storage devices.

With continued reference to FIG. 1, the example medical image management system environment 100 includes a general-purpose computing device in the form of a control server 110. Components of the control server 110 may include, without limitation, a processing unit, internal system memory, and a suitable system bus for coupling various system components, including a database cluster 120, with the control server 110. The system bus may be any of several types of bus structures, including a memory bus or memory controller, a peripheral bus, and a local bus, using any of a variety of bus architectures. By way of example, and not limitation, such architectures include the Industry Standard Architecture (ISA) bus, the Micro Channel Architecture (MCA) bus, the Enhanced ISA (EISA) bus, the Video Electronic Standards Association (VESA) local bus, and the Peripheral Component Interconnect (PCI) bus, also known as a Mezzanine bus.

The control server 110 may include therein, or have access to, a variety of computer-readable storage media (e.g., the database cluster 120). Computer-readable storage media can be any available media that may be accessed by the control server 110 and include volatile and non-volatile media, as well as removable and non-removable media. By way of example, and not limitation, computer-readable storage media may include computer storage media. Computer storage media may include, without limitation, volatile and non-volatile media, as well as removable and non-removable media implemented in any method or technology for storage of information, such as computer-readable instructions, data structures, program modules, or other data. In this regard, computer storage media may include, but are not limited to, random-access memory (RAM), read-only memory (ROM), electrically erasable programmable read-only memory (EEPROM), flash memory or other memory technology, compact disc read-only memory (CD-ROM), digital versatile disks (DVDs) or other optical disk storage, magnetic cassettes, magnetic tape, magnetic disk storage, or another magnetic storage device, or any other medium that can be used to store the desired information and may be accessed by the control server 110. By way of example, and not limitation, communication media include wired media such as a wired network or direct-wired connection and wireless media such as acoustic, radio frequency (RF), infrared, and other wireless media. Combinations of any of the above also may be included within the scope of computer-readable storage media.

The computer storage media discussed above and illustrated in FIG. 1, including the database cluster 120, provide storage of computer-readable instructions, data structures, program modules, and other data for the control server 110. The control server 110 may operate on a computer network 130 using logical connections to one or more remote computers (e.g., remote computer 140, remote computer 140', remote computer 140"). A remote computer may be located at a variety of locations in a medical or research environment, including, but not limited to, clinical laboratories (e.g., molecular diagnostic laboratories), hospitals and other inpatient settings, veterinary environments, ambulatory settings, medical billing and financial offices, hospital administration settings, home health care environments, and clinicians' offices. A remote computer may also be physically located in non-traditional medical care environments so that the entire healthcare community may be capable of integration into the network. Remote computers may be personal computers, servers, routers, network personal computers, peer devices, other common network nodes, or the like and may include some or all of the elements described above in relation to the control server 110. The devices can be personal digital assistants or other like devices.

An example computer network 130 may include, without limitation, local area networks (LANs) and/or wide area networks (WANs). Such networking environments are commonplace in offices, enterprise-wide computer networks, intranets, and the Internet. When utilized in a WAN networking environment, the control server 110 may include a modem or other means for establishing communications over the WAN, such as the Internet. In a networked environment, program modules or portions thereof may be stored in association with the control server 110, in association with the database cluster 120, or in association with one or more of the remote computers (e.g., remote computer 140). For example, and not by way of limitation, various application programs may reside on a memory associated with any one or more of the remote computers. It will be appreciated by those of ordinary skill in the art that the network connections shown are examples and other means of establishing a communications link between the computers (e.g., control server 110 and remote computer 140) may be utilized.

Clinicians may include, but are not limited to, a treating physician or physicians, specialists such as intensivists, surgeons, radiologists, cardiologists, and oncologists, emergency medical technicians, physicians' assistants, nurse practitioners, nurses, nurses' aides, pharmacists, dieticians, microbiologists, laboratory experts, laboratory technologists, radiologic technologists, researchers, veterinarians, students, and the like. In operation, a clinician may enter commands and information into the control server 110 or convey the commands and information to the control server 110 via one or more remote computers (e.g., remote computer 140, remote computer 140', remote computer 140") through input devices, such as a keyboard, a pointing device (commonly referred to as a mouse), a trackball, a touch pad, a touch screen, and the like. Other input devices may include, without limitation, microphones, scanners, motion sensors, and the like. Commands and information may also be sent directly from a remote healthcare device to the control server 110. A control server 110 and/or a remote computer (e.g., remote computer 140) may include other peripheral output devices, such as speakers and a printer.

Although many other internal components of the control server 110 and remote computers (e.g., remote computer 140, remote computer 140', remote computer 140") are not shown, those of ordinary skill in the art will appreciate that such components and their interconnection are well known. Accordingly, additional details concerning the internal construction of the control server 110 and remote computers are not further disclosed herein.

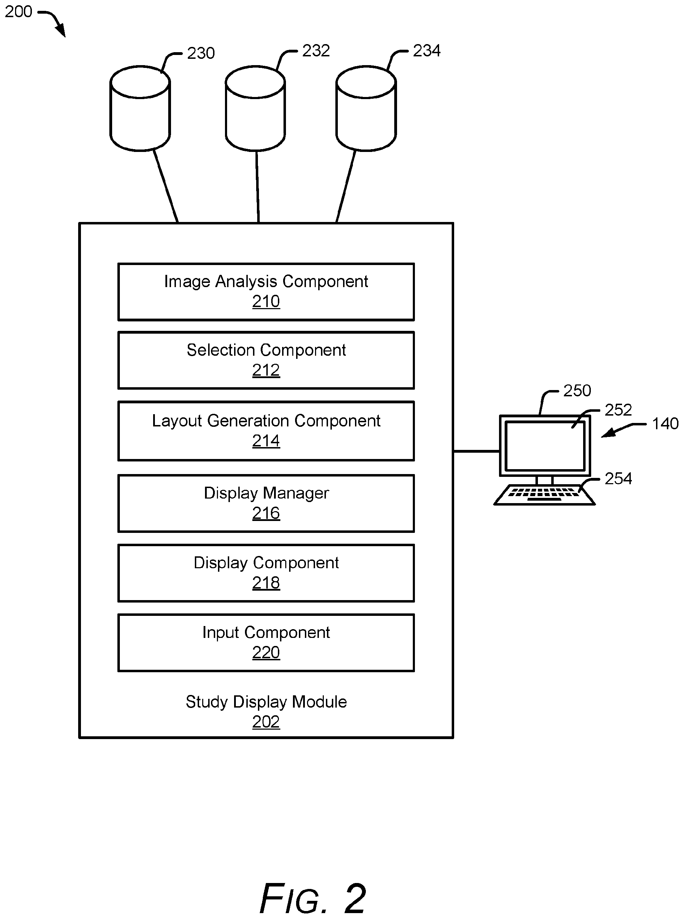

With reference to FIG. 2, a block diagram is illustrated that shows an example of a computing system architecture for displaying grouped medical images for review, for example, on a display device of a display component. It will be appreciated that the computing system architecture for a computing system 200 illustrated in FIG. 2 is merely an example of one suitable computing system and is not intended as having any dependency or requirement related to any single module/component or combination of modules/components.

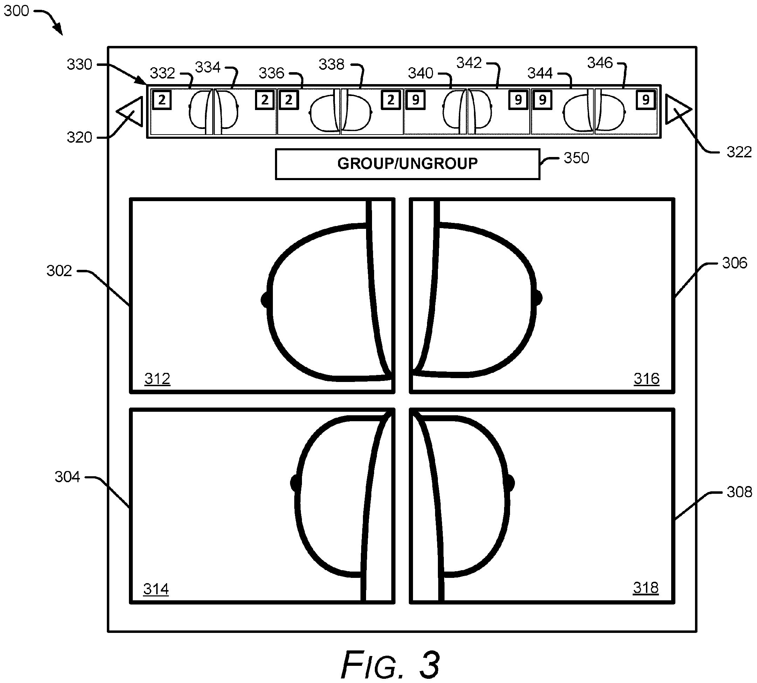

In one aspect, the computing system 200 includes a study display module 202 and one or more data sources (e.g., database cluster 120), which include at least one image data set of medical images for a healthcare study (e.g., projection X-ray images, projection X-ray mammographic images, computer-generated images, tomosynthesis images related to the projection X-ray images, technical repeat images, supplementary mammographic view images, CAD-generated images, thumbnail images, and the like). While, in the implementations of FIGs. 3-5, healthcare study information may be described and illustrated as including mammographic images, in other aspects the healthcare study information may include non-mammographic images.

In an example, the data sources may include at least one database 230 that stores and maintains current healthcare studies that contain current medical images, a database 232 that stores and maintains existing (previous) healthcare studies, and/or a database 234 that stores and maintains findings ("automated image analysis findings") that result from application of one or more automated image analysis algorithms (e.g., artificial intelligence (AI) analysis algorithms) to images of the current healthcare studies (e.g., those stored in database 230). A data source may contain images or other study data (e.g., medical parameter values) that are linked to a patient's electronic medical record (EMR). As utilized herein, the acronym "EMR" is not meant to be limiting and may broadly refer to any or all aspects of the patient's medical record rendered in a digital format. Generally, the EMR is supported by systems configured to co-ordinate the storage and retrieval of individual records with the aid of computing devices. As such, a variety of types of healthcare-related information may be stored and accessed in this way. One or more of the data sources may be maintained separately. Two or more of the data sources may be integrated. In aspects, the data sources (e.g., database 230, database 232, database 234) may be a picture archiving and communication system (PACS), a vendor neutral archive (VNA), other repository systems, or other database systems. The data sources may be spread across multiple facilities and/or multiple locations. A database may include one or more databases.

In one aspect, the healthcare studies include medical images and study data (e.g., healthcare study information). The healthcare study information may include values of one or more medical parameters (e.g., parameter values, measurements, findings, impressions, patient demographics and history/risk factors) related to the healthcare study. Medical images of a healthcare study may include radiology images (e.g., mammographic images), laboratory images, pictures, cardiology images (e.g., echocardiography images), and other healthcare images (e.g., medical image data). A healthcare study may include one or more series of one or more medical images, and the series of images may include one or more images that depict the subject of the image from various angles.

The computing system 200 includes a study display module 202, which includes one or more of an image analysis component 210, a selection component 212, a layout generation component 214, a display manager 216, a display component 218, and an input component 220. The study display module 202 may reside on one or more computing devices (e.g., control server 110 described above with reference to FIG. 1). By way of example, in one aspect, the control server 110 includes one or more computer processors and may be a server, personal computer, desktop computer, laptop computer, handheld device, mobile device, consumer electronic device, or the like.

The image analysis component 210 (e.g., an AI analysis component) may perform image analysis on healthcare studies (e.g., medical image data stored in database 230) to produce automated image analysis findings (e.g., computer-aided detection results). The automated image analysis findings may be sent for clinician review and/or may be stored in a data source (e.g., database 234) for subsequent access. The image analysis may include the activation of one or more automated image analysis algorithms that execute on one or more artificial intelligence (AI) engines and/or servers (e.g., control server 110 described above with reference to FIG. 1) to produce results that are indicative of the findings of the algorithm. The automated image analysis findings may include images, or portions thereof, from the analyzed healthcare study that are relevant to a diagnosis or condition of a patient. The image analysis component 210 may analyze medical imaging data and mark perceived latent features in the medical imaging data, for example, through use of one or more indicia (e.g., tags, icons, arrows, pointers) to generate annotated imaging data (e.g., CAD-generated images) for review by the clinician. In this way, the annotated imaging data may include a study image that is annotated with indicia to generate an annotated image, an overlay that includes indicia that are displayed on top of a study image, and the like.

The study display module 202 may receive healthcare study information (e.g., medical images) from a data source (e.g., a database, via a link within a patient's EMR). For example, the selection component 212 may select healthcare study information from a first data source and the study display module 202 may receive it. The healthcare study may include at least one of a current healthcare study or one or more previous healthcare studies. The healthcare study information may include an image data set of medical images for a healthcare study, which may include one or more series of one or more medical images. The healthcare study information may include annotated imaging data.

The selection component 212 is configured to select a healthcare study and may access data sources (e.g., databases) to receive it. A selected healthcare study may include one or more pieces of information (e.g., healthcare study information) related to the healthcare study. The healthcare study information may include, but is not limited or restricted to, (i) medical images (e.g., x-rays, mammograms, thumbnail images, computerized tomography (CT) scans, magnetic resonance imaging (MRI), positron emission tomography (PET) scans, ultrasound imaging), (ii) computer-aided detection results, (iii) clinician notes regarding one or more of the medical images, and/or (iv) medical records corresponding to one or more of the subjects of the one or more medical images. The medical records may include other study data, for example, medical parameter values (e.g., measurements, findings, impressions, patient demographics and history/risk factors) related to the healthcare study.

The selection component 212 may group one or more medical images together into a set. For example, the selection component 212 may group medical images for the imaging of a body part (e.g., breast) into a set by the same imaging position (e.g., CC, LMO) and side of the body (e.g., left, right). In an example, right CC images are grouped into a RCC group, left CC images are grouped into a LCC group, right MLO images are grouped into a RMLO group, and the like. In another example, the selection component 212 may group medical images for the imaging of the body part into a set that is based on images generated during a same compression sequence during a study (e.g., a same compression of the breast between a compression paddle and an imaging table). As a result of the grouping, the set-grouped mammographic images may include 2D images, supplementary mammographic views, images of a breast tomosynthesis series, synthetic images, technical repeat images, and/or CAD-generated images of the same imaging position and/or same side. The selection component 212 may extract features from image metadata (e.g., Digital Imaging and Communications in Medicine (DICOM) attributes, medical image data, image information received from an imaging device) to determine appropriate set grouping for a medical image.

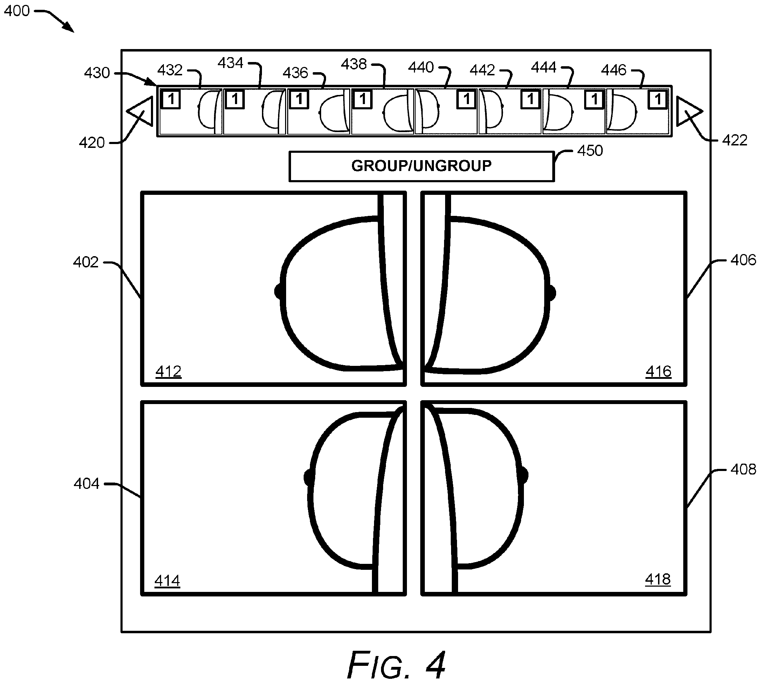

Utilizing the received healthcare study information, a layout generation component 214 may generate a display layout (e.g., layout) for display of mammographic images in a graphical user interface (GUI) on a display device of a display component 218. A display layout may include more than one type of projection to provide a side-by-side comparison of projections. In one example, illustrated in the aspects of FIG. 3 and FIG. 4, the display layout includes a 2x2 grid of four viewports for displaying healthcare study information (e.g., mammographic images). In such a configuration, the 2x2 grid includes an upper left viewport, a lower left viewport, an upper right viewport, and a lower right viewport. In such an example configuration, the images are stacked horizontally by imaging position (view) and are stacked vertically by same side (laterality).

A display layout may include the display of at least one thumbnail image configured to enable the clinician to navigate the contents of the healthcare study. The thumbnail image may be received from a data source (e.g., database cluster 120) and may be generated by the layout generation component 214 from the medical image data (e.g., mammographic images). When displayed in the GUI (e.g., within a user interface element, described below), a thumbnail image may include a numeric indicator that represents the total number of images in the set that the thumbnail represents.

The thumbnail image may be displayed in a user interface element (e.g., a thumbnail bar element, discussed below) of the GUI. The selection (e.g., by a clinician) of one or more thumbnail images may result in a change to the display layout, for example, the display of one or more medical images in the GUI (e.g., in a viewport of the GUI). For example, in a GUI that includes a 2x2 grid of four viewports, selection of four thumbnails that represent a first set (e.g., RCC), a second set (e.g., LCC), a third set (e.g., RMLO), and a fourth set (e.g., LMLO) results in the display of an image of the first set in the upper left viewport, an image of the second set in the upper right viewport, an image of the third set in the lower left viewport, and an image of the fourth set in the lower right viewport. A clinician may use drag-and-drop selection on a thumbnail image to trigger a comparison action. For example, the clinician may use a drag-and-drop selection of a thumbnail image to a viewport to cause the image or the image set represented by the thumbnail image to be displayed on the GUI (e.g., in one or more viewports) for comparison to another viewport display. To reverse such a comparison action, the clinician may drag the thumbnail image back to the user interface element of the GUI.

The layout generation component 214 utilizes the set grouping by the selection component 212 to generate a display layout for a grouped thumbnail image mode of the set-grouped thumbnail images for the current healthcare study. In a first implementation of a grouped thumbnail image mode, the layout generation component 214 generates a display layout that includes a grouping of the thumbnail images based on a grouping by the selection component 212 (e.g., grouping into a set by the same imaging position (e.g., CC, LMO) and side of the body (e.g., left, right)). In such an example, the thumbnail images displayed in the user interface element (e.g., a thumbnail bar element) may include right craniocaudal (CC) views (e.g., first and second RCC views) grouped together by a RCC thumbnail, right mediolateral oblique (MLO) views (e.g., first and second RMLO views) grouped together by a RMLO thumbnail, right mediolateral oblique (MLO) synthetic views (e.g., first and second RMLO synthetic views) grouped together by a RMLO synthetic view thumbnail, and the like. In a second implementation of a grouped thumbnail image mode, the display layout may include a further grouping of the side views (e.g., left and right) into the grouping by imaging position. For example, left and right craniocaudal (CC) views (e.g., first and second LCC and RCC views) may be grouped together by a LCC/RCC thumbnail, left and right mediolateral oblique (MLO) views (e.g., first and second LMLO and RMLO views) may be grouped together by a LMLO/RMLO thumbnail, left and right mediolateral oblique (MLO) synthetic views (e.g., first and second LMLO and RMLO synthetic views) may be grouped together by a LMLO/RMLO synthetic view thumbnail, and the like.

The layout generation component 214 further generates a display layout for an ungrouped thumbnail image mode. In the ungrouped thumbnail image mode, the display layout includes the display of ungrouped thumbnail images for the current healthcare study. For example, in the ungrouped thumbnail image mode, a first RCC thumbnail for a first RCC image, a second RCC thumbnail for a second RCC image, a first RMLO thumbnail for a first RMLO image, a second RMLO thumbnail for a second RMLO, and the like may be presented on the GUI. In the ungrouped thumbnail image mode, tomosynthesis series images may remain grouped together in set-grouped tomosynthesis images, for example, based on same imaging position and side.

A display layout generated by the layout generation component 214 may include a user interface element configured for display of the thumbnail images, referred to herein as a thumbnail bar element. In aspects, the thumbnail bar element may be one or more of an image slider, an image carousel, a carousel slider, a slider bar, a slide panel, a gallery, a slideshow, and the like. The thumbnail bar element may be configured to move (e.g., slide, rotate) automatically for navigation, may be configured for manual navigation (e.g., move from left to right) by a user without navigating away from the view of study on the GUI, etc. A user may interact with the thumbnail bar element to navigate through (e.g., scroll through) the thumbnail images (e.g., by swiping on a touchscreen display, pushing arrow keys on a keyboard, using a mouse).

The display layout may allow one or more images of the current study to be displayed for review by the clinician. The display layout may allow one or more images of the current study to be compared to one or more images of at least one previous study. The display layout may allow one or more images of the current study to be compared to one or more images (e.g., annotated imaging data) generated by the layout generation component 214 that include automated image analysis findings for the current study. In one aspect, the layout generation component 214 creates a side-by-side layout with images of the current and previously created studies rendered next to each other within the display component. The display layout may define configurations including parts of the screen layout, display contexts (e.g., image data, layouts, arrangement of images, size of images), and the like to facilitate an ordered review process.

The display manager 216 (e.g., rendering component) receives the display layout and generates a graphical user interface (GUI) on a display device of the display component 218. The GUI may be implemented within an image viewer or an image viewer application of the display device. The GUI may be configured to display contents of the current healthcare study, including any images and findings contained therein. The display component 218 includes a display device (e.g., a monitor, a computer screen, a project device, other hardware device) for displaying display layouts containing images and other data from healthcare studies in a GUI. For example, a remote computer 140 described above with reference to FIG. 1 may include a display component with a display device. The display component 218 displays the display layouts generated by the display manager 216 in the GUI of the display device, for example, in a GUI 252 of a display device 250 of the remote computer 140 illustrated in FIG. 2.

A display layout generated by the layout generation component 214 may further include a user interface element that enables user interaction with the GUI using an input component 220 (e.g., user interface input device). The input component 220 may receive a user input(s) from a user (e.g., a clinician) to change the user interface (e.g., display layout). For example, the input component 220 may receive user input from a user via a grouping selection user interface element to switch (e.g., toggle) the GUI (via the display manager 216) between the grouped and ungrouped thumbnail image modes (e.g., from a grouped thumbnail image mode to an ungrouped thumbnail image mode, from an ungrouped thumbnail image mode to a grouped thumbnail image mode). The user input may be received from a user interface input device (e.g., a keyboard, a keypad, a mouse, and the like). In one example, the input device is a keyboard 254 of the remote computer 140. The user input received by the input component 220 may include a command invoked by the clinician to change the user interface (e.g., display layout). Examples of a command invoked by a clinician include a button press, a keyboard press, a mouse click, a voice input, and the like. In one example, the command is a mouse click on a user interface element.

If the display layout is in a grouped thumbnail image mode, responsive to receiving a user input to change the user interface (e.g., display layout), the display manager 216 may switch the graphical user interface from the grouped thumbnail image mode to an ungrouped thumbnail image mode. If the display layout is in an ungrouped thumbnail image mode, responsive to receiving a user input to change the user interface (e.g., display layout), the display manager 216 may switch the graphical user interface from the ungrouped thumbnail image mode to a grouped thumbnail image mode.

FIG. 3 illustrates an example graphical user interface (GUI 300) that may be used in the system of FIGs. 1 and 2 for displaying grouped medical images for review, in accordance with some aspects. In portions of the following discussion, reference can be made to the example system environment 100 of FIG. 1 and/or to entities or processes as detailed in FIGs. 1 and 2, reference to which is made for example only. As discussed with respect to the computing system 200 of FIG. 2, a study display module (e.g., study display module 202) receives selected healthcare study information for a healthcare study from a data source. The healthcare study information is selected by a selection component (e.g., selection component 212). Utilizing the selected healthcare study information, a layout generation component (e.g., layout generation component 214) generates at least one display layout for displaying one or more of medical images (e.g., mammographic images) of a current healthcare study in a GUI (e.g., GUI 300) of a display device (e.g., display device 250 of remote computer 140). A display manager (e.g., display manager 216) receives the display layout(s) and generates the GUI on the display device, which displays the GUI to a clinician for review.

In FIG. 3, the example GUI 300 includes a 2x2 grid of four viewports for displaying the healthcare study information (e.g., mammographic images). In such a configuration, the 2x2 grid includes an upper left viewport 302, a lower left viewport 304, an upper right viewport 306, and a lower right viewport 308. In aspects, more or fewer viewports may be present for the medical images of the healthcare study. In the example configuration of FIG. 3, the images are stacked horizontally by imaging position (view) and are stacked vertically by same side (laterality). In the example GUI 300, the upper left viewport 302 displays a right craniocaudal (CC) view 312, the lower left viewport 304 displays a right mediolateral oblique (MLO) view 314, the upper right viewport 306 displays a left CC view 316, and the lower right viewport 308 displays a left MLO view 318 from the study. These are merely examples of projections that may be present in a display layout for the study displayed on an example GUI.

The GUI 300 includes the display of at least one thumbnail image in a thumbnail bar element 330. The presentation of the thumbnail image(s) enables the clinician to navigate the contents of the healthcare study. FIG. 3 illustrates a grouped thumbnail image mode wherein one or more of the medical images of the study may be grouped (e.g., by selection component 212) into a set, with each set represented by a thumbnail image displayed in the thumbnail bar element 330. In one example, where multiple medical images for the imaging of a body part (e.g., breast) are present, they may be grouped into a set by a same imaging position (e.g., CC, LMO) and side of the body (e.g., left, right). In this example, right mediolateral oblique (RMLO) views are grouped together by a RMLO thumbnail 332, left mediolateral oblique (LMLO) views are grouped together by a LMLO thumbnail 334, right craniocaudal (RCC) views are grouped together by a RCC thumbnail 336, left craniocaudal (LCC) views are grouped together by a LCC thumbnail 338, RMLO synthetic views are grouped together by a RMLO synthetic view thumbnail 340, LMLO synthetic views are grouped together by a LMLO synthetic view thumbnail 342, RCC synthetic views are grouped together by a RCC synthetic view thumbnail 344, LCC synthetic views are grouped together by a LMLO synthetic view thumbnail 346, and the like. Additional thumbnail groups may be present in the thumbnail bar element 330, but not visible, in the GUI 300 illustrated in FIG. 3. A navigational element (e.g., element 320, element 322) may be presented for navigation of the contents of the thumbnail bar element 330 (e.g., to access the additional thumbnail images). The images (e.g., views) grouped together may represent different series of images (e.g., a 2D image series, a tomosynthesis image series, a technical repeat series).

When displayed in the GUI 300, a thumbnail image may include a numeric indicator that represents the total number of images and/or series that are grouped into the set that the thumbnail represents. For example, the RMLO thumbnail 332 includes the numeric indicator "2," which indicates that the group includes a first RMLO image and a second RMLO image (e.g., a 2D image and a technical repeat). Likewise, the LMLO thumbnail 334, RCC thumbnail 336, and LCC thumbnail 338 include numeric indicators of "2" as well. In contrast, the RMLO synthetic view thumbnail 340, LMLO synthetic view thumbnail 342, RCC synthetic view thumbnail 344, and LMLO synthetic view thumbnail 346 each include numeric indicators of "9," indicating each thumbnail represents nine (9) synthetic tomosynthesis images.

The selection (e.g., by the clinician) of one or more thumbnail images may result in a change to the display layout. The selection of one or more thumbnails may result in the display of one or more images in the GUI (e.g., in a viewport of the GUI). For example, in a GUI that includes a 2x2 grid of four viewports (e.g., the GUI 300 of FIG. 3), the selection of four thumbnails that represent a first set (e.g., RCC), a second set (e.g., LCC), a third set (e.g., RMLO), and a fourth set (e.g., LMLO) results in the display of images of the first set in the upper left viewport, images of the second set in the upper right viewport, images of the third set in the lower left viewport, and images of the fourth set in the lower right viewport. If a set includes more than one image, a UI element (e.g., next page selector) may be presented to enable the clinician to scroll between images of the set.

During their review of the medical images displayed in the GUI 300, the clinician may provide (e.g., via input component 220) user input to change the GUI. For example, providing input on a grouping selection user interface element 350 (e.g., a button in the GUI) to switch the GUI between the grouped thumbnail image mode and an ungrouped thumbnail image mode (e.g., from a grouped thumbnail image mode to an ungrouped thumbnail image mode, from an ungrouped thumbnail image mode to a grouped thumbnail image mode). If the display layout is in a grouped thumbnail image mode, responsive to receiving a user input to change the user interface, the display manager (e.g., display manager 216) may switch the GUI from a grouped thumbnail image mode (illustrated in FIG. 3) to an ungrouped thumbnail image mode (illustrated in FIG. 4).

FIG. 4 illustrates an example graphical user interface (GUI 400) that may be used in the system of FIGs. 1 and 2 for displaying grouped medical images for review, in accordance with some aspects. In portions of the following discussion, reference can be made to the example system environment 100 of FIG. 1 and/or to entities or processes as detailed in FIGs. 1 and 2, reference to which is made for example only. As discussed with respect to the computing system 200 of FIG. 2, a study display module (e.g., study display module 202) receives selected healthcare study information for a healthcare study from a data source. The healthcare study information is selected by a selection component (e.g., selection component 212).

Utilizing the selected healthcare study information, a layout generation component (e.g., layout generation component 214) generates at least one display layout for displaying one or more of medical images (e.g., mammographic images) of a current healthcare study in a GUI of a display device (e.g., display device 250 of remote computer 140). A display manager (e.g., display manager 216) receives the display layout(s) and generates the GUI on the display device, which displays the GUI to a clinician for review. The GUI 400 is similar to the GUI 300 illustrated in FIG. 3 and described above, except as detailed below. Thus, the GUI 400 includes an upper left viewport 402, a lower left viewport 404, an upper right viewport 406, a lower right viewport 408, a navigational element 420, a navigational element 422, a thumbnail bar element 430, and a grouping selection user interface element 450. In the example GUI 400, the upper left viewport 402 displays a right craniocaudal (CC) view 412, the lower left viewport 404 displays a right mediolateral oblique (MLO) view 414, the upper right viewport 406 displays a left CC view 416, and the lower right viewport 408 displays a left MLO view 418 from the study.

FIG. 4 illustrates an ungrouped thumbnail image mode wherein a thumbnail image displayed in the thumbnail bar element 430 does not reflect set-grouped (e.g., by selection component 212) medical images of the study. In such a mode, where multiple medical images for the imaging of a body part (e.g., breast) are present, they may not be grouped into a set by the same imaging position (e.g., CC, LMO) and side of the body (e.g., left, right). In this example, right mediolateral oblique (RMLO) views are not grouped and are represented by a first RMLO thumbnail 432 and second RMLO thumbnail 434, left mediolateral oblique (LMLO) views are not grouped and are represented by a first LMLO thumbnail 436 and second LMLO thumbnail 438, right craniocaudal (RCC) views are not grouped and are represented by a first RCC thumbnail 440 and second RCC thumbnail 442, and left craniocaudal (LCC) views are not grouped and are represented by a first LCC thumbnail 444 and second LCC thumbnail 446. Additional thumbnails and/or thumbnail groups may be present in the thumbnail bar element 430, but not visible, in the GUI 400 illustrated in FIG. 4.

When displayed in the GUI 400, a thumbnail image may include a numeric indicator that represents the total number of images and/or series that are grouped into the set that the thumbnail represents. For example, the first RMLO thumbnail 432, second RMLO thumbnail 434, first LMLO thumbnail 436, second LMLO thumbnail 438, first RCC thumbnail 440, second RCC thumbnail 442, first LCC thumbnail 444, and second LCC thumbnail 446 each include the numeric indicator "1," which indicates that the group (image) represented by the thumbnail includes a single image.

During their review of the medical images displayed in the GUI 400, the clinician may provide (e.g., via input component 220) user input to change the GUI. For example, the clinician may provide input on the grouping selection user interface element 450 (e.g., a button in the GUI) to switch the GUI between the ungrouped thumbnail image mode and a grouped thumbnail image mode (e.g., from a grouped thumbnail image mode to an ungrouped thumbnail image mode, from an ungrouped thumbnail image mode to a grouped thumbnail image mode). If the display layout is in an ungrouped thumbnail image mode, responsive to receiving a user input to change the user interface (e.g., display layout), the display manager 216 may switch the GUI from an ungrouped thumbnail image mode (illustrated in FIG. 4) to a grouped thumbnail image mode (illustrated in FIG. 3). In aspects, sets of synthetic tomosynthesis images (e.g., RMLO synthetic views, LMLO synthetic views, RCC synthetic views, LCC synthetic views) may not be ungrouped by selection of the grouping selection user interface element 450.

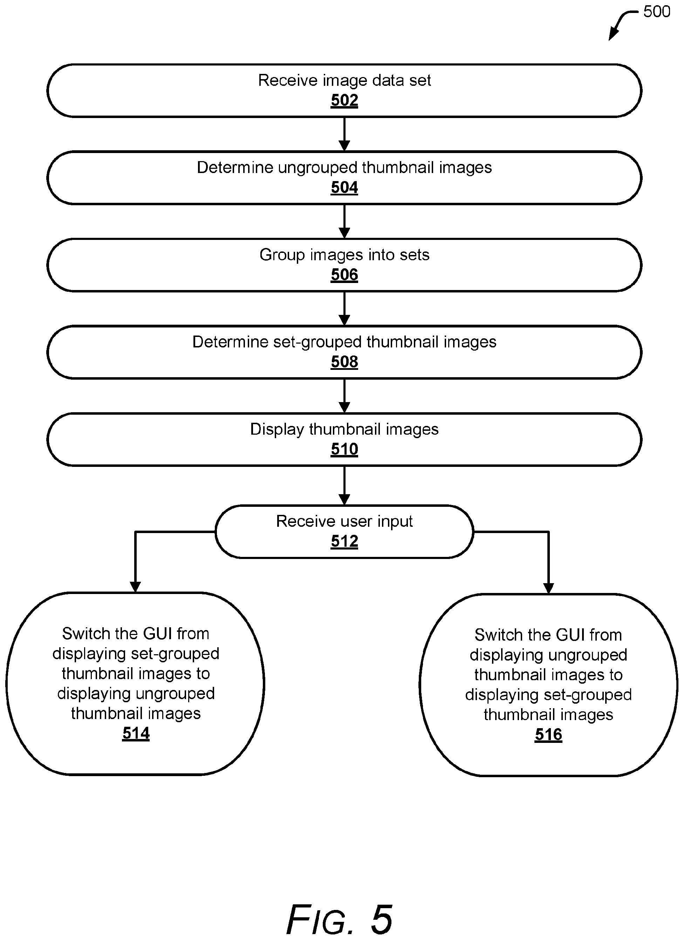

FIG. 5 is a flow diagram of one implementation of a process 500 for displaying grouped medical images for review performed by a medical image management system (e.g., medical image management system environment 100 of FIG. 1, medical image management system 600). The process 500 is performed by processing logic that may include hardware (e.g., circuitry, dedicated logic), software (e.g., software run on a general-purpose computer system, software run on a dedicated machine), firmware, or a combination of two or more of these. The medical image management system may include a network communication interface that is configured to receive images of a healthcare study; an image cache memory to cache the images received; one or more processors coupled to the network connection interface and the memory and configured to display healthcare study information; and a display component (e.g., display screen) coupled to the one or more processors to display the images in a graphical user interface (GUI) of a display device.

The process 500 is shown as a set of blocks that specify operations performed but are not necessarily limited to the order or combinations shown for performing the operations by the respective blocks. Further, any of one or more of the operations can be repeated, combined, reorganized, or linked to provide a wide array of additional and/or alternate methods. In portions of the following discussion, reference can be made to the example system environment 100 of FIG. 1 and/or to entities or processes as detailed in FIGs. 1-4, reference to which is made for example only. The techniques are not limited to performance by one entity or multiple entities operating on one device.

Referring to FIG. 5, the process 500 begins with the processing logic receiving an image data set (e.g., medical images of a healthcare study) from a data source (processing block 502). The image data set may include projection X-ray images (e.g., projection X-ray mammographic images) for the healthcare study. In aspects, the image data set is sent to the medical image management system by a modality that creates the study. In aspects, the image data set is sent from a medical image archive (e.g., a picture archiving and communication system (PACS) or other remotely located storage facility). In aspects, the image data set is received via a network interface and stored in a memory of the medical image management system. The processing logic determines an ungrouped thumbnail image for each image (e.g., projection X-ray image, projection X-ray mammographic image) of the image data set (processing block 504). In aspects, the ungrouped thumbnail image is determined by receiving it from the data source. The ungrouped thumbnail images are for display in a graphical user interface of a display device.

The processing logic groups images of the image data set into a plurality of sets (processing block 506). The processing logic determines a set-grouped thumbnail image for each set of grouped images (processing block 508). The set-grouped thumbnail images are configured for display in the graphical user interface. The processing logic generates a graphical user interface that includes displaying at least one of the set-grouped thumbnail images or the ungrouped thumbnail images in the graphical user interface (processing block 510). The processing logic receives user input on a user interface element (processing block 512). A grouping selection user interface element may be displayed in the graphical user interface to receive the user input. The grouping selection user interface element may be configured for switching the graphical user interface from a grouped thumbnail image mode to an ungrouped thumbnail image mode. **In** response to receiving user input on the user interface element, the processing logic (1) switches the graphical user interface from displaying set-grouped thumbnail images in the graphical user interface to displaying ungrouped thumbnail images in the graphical user interface (processing block 514) or (2) switches the graphical user interface from displaying ungrouped thumbnail images in the graphical user interface to displaying set-grouped thumbnail images in the graphical user interface (processing block 516).

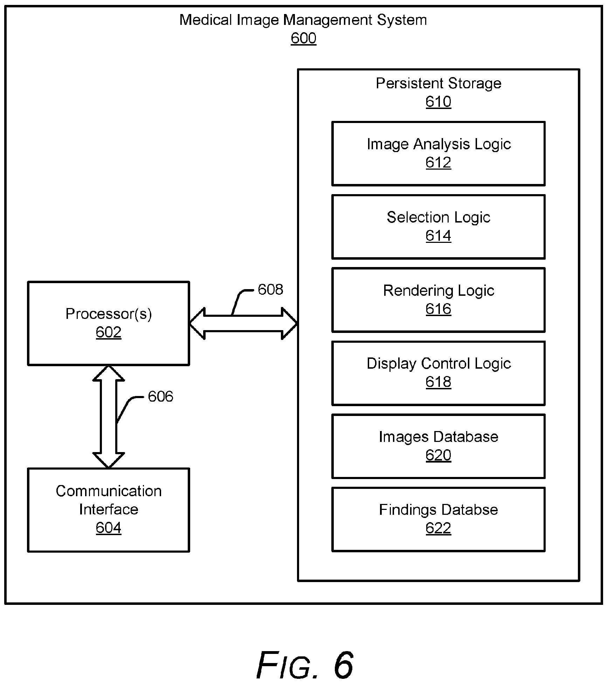

FIG. 6 illustrates an example aspect of a logical representation of a medical image management system 600 for displaying grouped medical images for review that is discussed above. The medical image management system 600 may be implemented by one or more of the control server 110, a remote computer (e.g., remote computer 140), and/or the database cluster 120 of the medical image management system environment 100 discussed above with respect to FIG. 1. The medical image management system 600 includes one or more processors 602 that are coupled to communication interface logic 604 via a first transmission medium 606. The communication interface logic 604 enables communications with other electronic devices, specifically enabling communication with remote users such as doctors, nurses and/or medical technicians, remote databases (e.g., PACS) that store healthcare studies, and healthcare modalities that generate and send studies. The communication interface logic 604 may be implemented as a physical interface including one or more ports for wired connectors. Additionally, or in the alternative, the communication interface logic 604 may be implemented with one or more radio units for supporting wireless communications with other electronic devices.

The medical image management system 600 performs a portion or all of the processing steps of systems and methods for displaying grouped medical images for review in response to processor(s) 602 executing one or more sequences of one or more instructions contained in a memory, such as persistent storage 610. Such instructions may be read into the persistent storage 610 from another computer-readable medium, such as a storage device. One or more processors in a multi-processing arrangement may also be employed to execute the sequences of instructions contained in the persistent storage 610. In aspects, hard-wired circuitry may be used in place of or in combination with software instructions. Thus, aspects are not limited to any specific combination of hardware circuitry and software.

As stated above, the medical image management system 600 includes at least one computer-readable medium or memory programmed according to the teachings of the disclosed systems and methods for displaying grouped medical images for review and for containing data structures, tables, records, or other data described herein. Examples of computer-readable storage media are compact discs, hard disks, floppy disks, tape, magneto-optical disks, PROMs (erasable programmable read-only memory (EPROM), EEPROM, Flash EPROM), dynamic random-access memory, static random-access memory, synchronous DRAM, and the like. Stored on any one or on a combination of computer-readable storage media, the disclosed systems and methods for displaying grouped medical images for review include software for controlling the medical image management system 600, for driving a device or devices for implementing disclosed systems and methods, and for enabling the medical image management system 600 to interact with a human user. Such software may include, but is not limited to, device drivers, operating systems, development tools, and applications software. Such computer-readable storage media further include a computer program product of the disclosed systems and methods for performing all or a portion (if processing is distributed) of the processing performed in implementing the disclosed systems and methods for displaying grouped medical images for review. Computer code devices of the disclosed systems and methods for displaying grouped medical images for review may be any interpreted or executable code mechanism, including but not limited to scripts, interpreters, dynamic link libraries, Java classes, and complete executable programs. Moreover, parts of the processing of the disclosed systems and methods for displaying grouped medical images for review may be distributed for better performance, reliability, and/or cost. The term "computer-readable medium," as used herein, refers to any medium that participates in providing instructions to processors 602 for execution. A computer-readable medium may take many forms, including but not limited to non-volatile media, volatile media, and transmission media.

The processor(s) 602 are further coupled to the persistent storage 610 via a transmission medium 608. The persistent storage 610 may store information and instructions to be executed by the processors 602. In addition, the persistent storage 610 may be used for storing temporary variables or other intermediate information during execution of instructions to be executed by processors 602. Persistent storage 610 may include (a) image analysis logic 612, (b) selection logic 614, (c) rendering logic 616, (d) display control logic 618, (e) an images database 620, and (f) a findings database 622.

Image analysis logic 612 includes logic for performing analysis of images from healthcare studies (e.g., medical images). The image analysis may include activating one or more automated image analysis algorithms executing on one or more artificial intelligence (AI) engines and/or servers to produce findings (e.g., results, outputs, annotated imaging data) that are indicative of the results of the algorithm. The findings may include images, or portions thereof, from the analyzed healthcare study that are relevant to a diagnosis or condition of a patient. Image analysis logic may perceive and mark latent features within medical imaging data (e.g., study images) to generate annotated imaging data. The image analysis logic may utilize one or more indicia (e.g., tags, icons, arrows, pointers) to mark the latent features in the medical imaging data. The annotated imaging data may include a study image that is annotated with indicia to generate an annotated image, an overlay including indicia that is displayed on top of a study image, and the like. The annotated imaging data may be stored in a data source.

The selection logic 614 may include logic for selecting healthcare studies (e.g., an image data set of medical images, thumbnail images) and accessing data sources (e.g., databases) to obtain the selected healthcare studies (e.g., retrieving healthcare study information from a storage device for display in a graphical user interface). The healthcare study information may include, but is not limited or restricted to, (i) medical images, including x-rays, mammograms, thumbnail images, CT scans, MRI, PET scans, and/or ultrasound imaging, (ii) computer-aided detection results, (iii) clinician notes regarding one or more of the medical images, and/or (iv) medical records corresponding to one or more of subjects of the one or more medical images. The medical records may include other study data, for example medical parameter values (e.g., measurements, findings, impressions, patient demographics and history/risk factors) related to the healthcare study. In an example, the healthcare study information may include a first image data set that includes projection X-ray mammographic images and thumbnail images for a healthcare study and a second image data set that includes tomosynthesis projection images related to the projection X-ray mammographic images of the study. The first image data set may be received from a first image data source and the second image data set may be received from a second image data source. The first and second data sources may be the same data source or may be different data sources. The selection logic 614 may include logic for determining an ungrouped thumbnail image for each image (e.g., projection X-ray image) of the image data set. The selection logic 614 may include logic for grouping images of the first and/or second image data set into a plurality of sets. The selection logic 614 may include logic for thumbnail image sets of grouped images.

The rendering logic 616 includes logic for generating data for graphical user interfaces (e.g., those described above). In one aspect, the rendering logic 616 includes logic for generating a graphical user interface that includes displaying thumbnail images in the graphical user interface, for example, in a thumbnail bar element configured to display thumbnail images.

The display control logic 618 receives data generated for graphical user interfaces and renders the graphical user interfaces on the display. The display control logic 618 includes logic for displaying healthcare study information in the graphical user interface. The display control logic 618 includes logic for receiving user input on a user interface element. **In** aspects, in response to receiving user input on the user interface element, the display control logic 618 includes logic for switching the graphical user interface from displaying set-grouped thumbnail images in the graphical user interface to displaying ungrouped thumbnail images in the graphical user interface. In aspects, in response to receiving user input on the user interface element, the display control logic 618 includes logic for switching (e.g., modifying) the graphical user interface from displaying ungrouped thumbnail images in the graphical user interface to displaying set-grouped thumbnail images in the graphical user interface.

The images database 620 and the findings database 622 may include a single non-transitory computer-readable medium storage device or may each be a separate non-transitory computer-readable medium storage device. The images database 620 stores healthcare study information (e.g., medical images) for display in a display area of an image viewer or other GUI. The findings database 622 stores automated image analysis findings (e.g., images) for display in a display area of an image viewer or other GUI.

Some additional examples of systems and methods for displaying grouped medical images for review include the following Examples.

There is provided a method comprising: receiving an image data set from a data source, the image data set including projection X-ray mammographic images for a healthcare study; determining an ungrouped thumbnail image for each projection X-ray mammographic image of the image data set, the ungrouped thumbnail images for display in a graphical user interface of a display device; grouping images of the image data set into a plurality of sets; determining a set-grouped thumbnail image for each set of grouped images, the set-grouped thumbnail images for display in the graphical user interface; generating a graphical user interface that includes displaying at least one of the set-grouped thumbnail images or the ungrouped thumbnail images in the graphical user interface; receiving user input on a user interface element; and, in response to receiving user input on the user interface element: switching the graphical user interface from displaying set-grouped thumbnail images in the graphical user interface to displaying ungrouped thumbnail images in the graphical user interface; or switching the graphical user interface from displaying ungrouped thumbnail images in the graphical user interface to displaying set-grouped thumbnail images in the graphical user interface.

The method may comprise: selecting projection X-ray mammographic images from the image data set for display in the graphical user interface; and displaying the selected projection X-ray mammographic images in the graphical user interface.

Generating a graphical user interface that includes displaying at least one of the set-grouped thumbnail images or the ungrouped thumbnail images in the graphical user interface may comprise: generating a thumbnail bar element, the thumbnail bar element configured to display at least one of the ungrouped thumbnail images or the set-grouped thumbnail images.

Switching the graphical user interface from displaying set-grouped thumbnail images in the graphical user interface to displaying ungrouped thumbnail images in the graphical user interface may comprise: modifying the generated graphical user interface to display the ungrouped thumbnail images in the thumbnail bar element in the graphical user interface; and displaying at least one of the ungrouped thumbnail images in the thumbnail bar element.

Switching the graphical user interface from displaying ungrouped thumbnail images in the graphical user interface to displaying set-grouped thumbnail images in the graphical user interface may comprise: modifying the generated graphical user interface to display the set-grouped thumbnail images in the thumbnail bar element in the graphical user interface; and displaying at least one of the set-grouped thumbnail images in the thumbnail bar element.

Optionally, the image data set is a first image data set, and the thumbnail bar element is configured to display tomosynthesis thumbnail images, and the method may comprise: receiving a second image data set from a data source, the second image data set including tomosynthesis projection images related to the projection X-ray mammographic images of the first image data set; grouping images of the second image data set into at least one tomosynthesis projection image series; and determining, for each tomosynthesis projection image series, a tomosynthesis thumbnail image for display in the graphical user interface, wherein generating the graphical user interface comprises: displaying at least one tomographic thumbnail image in the thumbnail bar element in the graphical user interface.

Switching the graphical user interface from displaying set-grouped thumbnail images in the graphical user interface to displaying ungrouped thumbnail images in the graphical user interface may not cause a display of ungrouped tomosynthesis projection image series in the thumbnail bar element in the graphical user interface.

The image data set may be a first image data set, and the data source for the first image data set and the data source for the second image data set may be a same data source.

Grouping images of the image data set into a plurality of sets may comprise grouping images of the image data set based on at least one of: imaging position and side; or compression and side. Determining a thumbnail image for each set of grouped images may comprise: generating a thumbnail image that shows a grouping based on imaging position and side. Generating the graphical user interface that includes displaying the set-grouped thumbnail images in the graphical user interface may comprise: displaying the set-grouped thumbnail images that show a grouping based on laterality and views in the graphical user interface.

There is provided, a medical image management system comprising: a network communication interface to receive healthcare studies; a memory coupled to the network communication interface to store received healthcare studies; a display screen coupled to the memory to display the received healthcare studies; and one or more processors coupled to the network communication interface, the memory, and the display screen and configured to: receive an image data set from a data source, the image data set including projection X-ray mammographic images for a healthcare study; determine an ungrouped thumbnail image for each projection X-ray mammographic image of the image data set, the ungrouped thumbnail images for display in a graphical user interface of a display device; group images of the image data set into a plurality of sets; determine a set-grouped thumbnail image for each set of grouped images, the set-grouped thumbnail images for display in the graphical user interface; generate a graphical user interface that includes displaying at least one of the set-grouped thumbnail images or the ungrouped thumbnail images in the graphical user interface; receive user input on a user interface element; and, in response to receiving user input on the user interface element: switch the graphical user interface from displaying set-grouped thumbnail images in the graphical user interface to displaying ungrouped thumbnail images in the graphical user interface; or switch the graphical user interface from displaying ungrouped thumbnail images in the graphical user interface to displaying set-grouped thumbnail images in the graphical user interface.

The one or more processors may be configured to: select projection X-ray mammographic images from the image data set for display in the graphical user interface; and display the selected projection X-ray mammographic images in the graphical user interface.

The one or more processors configured to generate a graphical user interface that includes displaying at least one of the set-grouped thumbnail images or the ungrouped thumbnail images in the graphical user interface may be configured to: generate a thumbnail bar element, the thumbnail bar element configured to display at least one of the ungrouped thumbnail images or the set-grouped thumbnail images.

The one or more processors configured to switch the graphical user interface from displaying set-grouped thumbnail images in the graphical user interface to displaying ungrouped thumbnail images in the graphical user interface may be configured to: modify the generated graphical user interface to display the ungrouped thumbnail images in the thumbnail bar element in the graphical user interface; and display at least one of the ungrouped thumbnail images in the thumbnail bar element.

The one or more processors configured to switch the graphical user interface from displaying ungrouped thumbnail images in the graphical user interface to displaying set-grouped thumbnail images in the graphical user interface may be configured to: modify the generated graphical user interface to display the set-grouped thumbnail images in the thumbnail bar element in the graphical user interface; and display at least one of the set-grouped thumbnail images in the thumbnail bar element.

There is provided, a non-transitory computer-readable storage media having instructions stored thereupon that when executed by a system having at least one processor, a memory, and a display screen therein, cause the system to perform a method comprising: receiving an image data set from a data source, the image data set including projection X-ray mammographic images for a healthcare study; determining an ungrouped thumbnail image for each projection X-ray mammographic image of the image data set, the ungrouped thumbnail images for display in a graphical user interface of a display device; grouping images of the image data set into a plurality of sets; determining a set-grouped thumbnail image for each set of grouped images, the set-grouped thumbnail images for display in the graphical user interface; generating a graphical user interface that includes displaying at least one of the set-grouped thumbnail images or the ungrouped thumbnail images in the graphical user interface; receiving user input on a user interface element; and, in response to receiving user input on the user interface element: switching the graphical user interface from displaying set-grouped thumbnail images in the graphical user interface to displaying ungrouped thumbnail images in the graphical user interface; or switching the graphical user interface from displaying ungrouped thumbnail images in the graphical user interface to displaying set-grouped thumbnail images in the graphical user interface.

The instructions may cause the system to perform: selecting projection X-ray mammographic images from the image data set for display in the graphical user interface; and displaying the selected projection X-ray mammographic images in the graphical user interface.

The instructions that cause the system to perform a method comprising generating a graphical user interface that includes displaying at least one of the set-grouped thumbnail images or the ungrouped thumbnail images in the graphical user interface may cause the system to perform: generating a thumbnail bar element, the thumbnail bar element configured to display at least one of the ungrouped thumbnail images or the set-grouped thumbnail images.

The instructions that cause the system to perform a method comprising switching the graphical user interface from displaying set-grouped thumbnail images in the graphical user interface to displaying ungrouped thumbnail images in the graphical user interface may cause the system to perform: modifying the generated graphical user interface to display the ungrouped thumbnail images in the thumbnail bar element in the graphical user interface; and displaying at least one of the ungrouped thumbnail images in the thumbnail bar element.

The instructions that cause the system to perform a method comprising switching the graphical user interface from displaying ungrouped thumbnail images in the graphical user interface to displaying set-grouped thumbnail images in the graphical user interface may cause the system to perform: modifying the generated graphical user interface to display the set-grouped thumbnail images in the thumbnail bar element in the graphical user interface; and displaying at least one of the set-grouped thumbnail images in the thumbnail bar element.

Optionally, the image data set is a first image data set, the thumbnail bar element is configured to display tomosynthesis thumbnail images, and the instructions may cause the system to perform: receiving a second image data set from a data source, the second image data set including tomosynthesis projection images related to the projection X-ray mammographic images of the first image data set; grouping images of the second image data set into at least one tomosynthesis projection image series; and determining, for each tomosynthesis projection image series, a tomosynthesis thumbnail image for display in the graphical user interface, and wherein generating the graphical user interface comprises: displaying at least one tomographic thumbnail image in the thumbnail bar element in the graphical user interface.

The disclosed systems and methods for displaying grouped medical images for review are advantageous, as they enable clinicians to quickly find medical image data (e.g., mammographic images) they need to complete their review, thereby reducing the amount of time required for review, which improves the clinician's user experience, enables the clinician to work more efficiently, and improves the timeliness of patient care.

The following clauses set out features of the invention which may or may not presently be claimed but which may form the basis for future amendments and/or divisional applications:
1. A non-transitory computer-readable storage media having instructions stored thereupon that when executed by a system having at least one processor, a memory, and a display screen therein, cause the system to perform a method comprising:
   receiving an image data set from a data source, the image data set including projection X-ray mammographic images for a healthcare study;
   determining an ungrouped thumbnail image for each projection X-ray mammographic image of the image data set, the ungrouped thumbnail images for display in a graphical user interface of a display device;
   grouping images of the image data set into a plurality of sets;
   determining a set-grouped thumbnail image for each set of grouped images, the set-grouped thumbnail images for display in the graphical user interface;
   generating a graphical user interface that includes displaying at least one of the set-grouped thumbnail images or the ungrouped thumbnail images in the graphical user interface;
   receiving user input on a user interface element; and,
   in response to receiving user input on the user interface element:
      switching the graphical user interface from displaying set-grouped thumbnail images in the graphical user interface to displaying ungrouped thumbnail images in the graphical user interface; or
      switching the graphical user interface from displaying ungrouped thumbnail images in the graphical user interface to displaying set-grouped thumbnail images in the graphical user interface.
**2.** The computer-readable storage media of clause 1, wherein the instructions cause the system to further perform:
   selecting projection X-ray mammographic images from the image data set for display in the graphical user interface; and
   displaying the selected projection X-ray mammographic images in the graphical user interface.
**3.** The computer-readable storage media of clause 1, wherein the instructions that cause the system to perform a method comprising generating a graphical user interface that includes displaying at least one of the set-grouped thumbnail images or the ungrouped thumbnail images in the graphical user interface further cause the system to perform:
   generating a thumbnail bar element, the thumbnail bar element configured to display at least one of the ungrouped thumbnail images or the set-grouped thumbnail images.
**4.** The computer-readable storage media of clause 3, wherein the instructions that cause the system to perform a method comprising switching the graphical user interface from displaying set-grouped thumbnail images in the graphical user interface to displaying ungrouped thumbnail images in the graphical user interface further cause the system to perform:
   modifying the generated graphical user interface to display the ungrouped thumbnail images in the thumbnail bar element in the graphical user interface; and
   displaying at least one of the ungrouped thumbnail images in the thumbnail bar element.
**5.** The computer-readable storage media of clause 3, wherein the instructions that cause the system to perform a method comprising switching the graphical user interface from displaying ungrouped thumbnail images in the graphical user interface to displaying set-grouped thumbnail images in the graphical user interface further cause the system to perform:
   modifying the generated graphical user interface to display the set-grouped thumbnail images in the thumbnail bar element in the graphical user interface; and
   displaying at least one of the set-grouped thumbnail images in the thumbnail bar element.
**6.** The computer-readable storage media of clause 3, wherein the image data set is a first image data set, wherein the thumbnail bar element is further configured to display tomosynthesis thumbnail images, wherein the instructions further cause the system to perform:
   receiving a second image data set from a data source, the second image data set including tomosynthesis projection images related to the projection X-ray mammographic images of the first image data set;
   grouping images of the second image data set into at least one tomosynthesis projection image series; and
   determining, for each tomosynthesis projection image series, a tomosynthesis thumbnail image for display in the graphical user interface, and
   wherein generating the graphical user interface further comprises:
      displaying at least one tomographic thumbnail image in the thumbnail bar element in the graphical user interface.

## Claims

1. A method (500) comprising:
receiving (502) an image data set from a data source, the image data set including projection X-ray mammographic images for a healthcare study;
determining (504) an ungrouped thumbnail image for each projection X-ray mammographic image of the image data set, the ungrouped thumbnail images for display in a graphical user interface (300; 400) of a display device;
grouping (506) images of the image data set into a plurality of sets;
determining (508) a set-grouped thumbnail image for each set of grouped images, the set-grouped thumbnail images for display in the graphical user interface (300; 400);
generating a graphical user interface (300; 400) that includes displaying at least one of the set-grouped thumbnail images or the ungrouped thumbnail images in the graphical user interface (300; 400);
receiving (512) user input on a user interface element; and,
in response to receiving user input on the user interface element:
switching (514) the graphical user interface (300; 400) from displaying set-grouped thumbnail images in the graphical user interface (300; 400) to displaying ungrouped thumbnail images in the graphical user interface (300; 400); or
switching (516) the graphical user interface (300; 400) from displaying ungrouped thumbnail images in the graphical user interface (300; 400) to displaying set-grouped thumbnail images in the graphical user interface (300; 400).

2. The method (500) of claim 1, further comprising:
selecting projection X-ray mammographic images from the image data set for display in the graphical user interface (300;400); and
displaying the selected projection X-ray mammographic images in the graphical user interface (300; 400).

3. The method (500) of claim 1 or 2, wherein generating a graphical user interface (300; 400) that includes displaying at least one of the set-grouped thumbnail images or the ungrouped thumbnail images in the graphical user interface (300; 400) further comprises:
generating a thumbnail bar element (330; 430), the thumbnail bar element (330; 430) configured to display at least one of the ungrouped thumbnail images or the set-grouped thumbnail images.

4. The method (500) of claim 3, wherein switching the graphical user interface (300; 400) from displaying set-grouped thumbnail images in the graphical user interface (300; 400) to displaying ungrouped thumbnail images in the graphical user interface (300; 400) comprises:
modifying the generated graphical user interface (300; 400) to display the ungrouped thumbnail images in the thumbnail bar element (330; 430) in the graphical user interface (300; 400); and
displaying at least one of the ungrouped thumbnail images in the thumbnail bar element (330; 430).

5. The method (500) of claim 3 or 4, wherein switching the graphical user interface (300; 400) from displaying ungrouped thumbnail images in the graphical user interface (300; 400) to displaying set-grouped thumbnail images in the graphical user interface (300; 400) comprises:
modifying the generated graphical user interface (300; 400) to display the set-grouped thumbnail images in the thumbnail bar element (330; 430) in the graphical user interface (300; 400); and
displaying at least one of the set-grouped thumbnail images in the thumbnail bar element (330; 430).

6. The method (500) of any of claims 3 to 5, wherein the image data set is a first image data set, and wherein the thumbnail bar element (330; 430) is further configured to display tomosynthesis thumbnail images, the method further comprising:
receiving a second image data set from a data source, the second image data set including tomosynthesis projection images related to the projection X-ray mammographic images of the first image data set;
grouping images of the second image data set into at least one tomosynthesis projection image series; and
determining, for each tomosynthesis projection image series, a tomosynthesis thumbnail image for display in the graphical user interface,
wherein generating the graphical user interface (300; 400) further comprises:
displaying at least one tomographic thumbnail image in the thumbnail bar element (330; 430) in the graphical user interface (300; 400).

7. The method (500) of claim 6, wherein switching the graphical user interface (300; 400) from displaying set-grouped thumbnail images in the graphical user interface (300; 400) to displaying ungrouped thumbnail images in the graphical user interface (300; 400) does not cause a display of ungrouped tomosynthesis projection image series in the thumbnail bar element (330; 430) in the graphical user interface (300; 400).

8. The method (500) of claim 6 or 7, wherein the image data set is a first image data set, and wherein the data source for the first image data set and the data source for the second image data set are a same data source.

9. The method (500) of any preceding claim,
wherein grouping images of the image data set into a plurality of sets further comprises grouping images of the image data set based on at least one of:
imaging position and side; or
compression and side;
wherein determining a thumbnail image for each set of grouped images further comprises:
generating a thumbnail image that shows a grouping based on imaging position and side; and
wherein generating the graphical user interface (300; 400) that includes displaying the set-grouped thumbnail images in the graphical user interface (300; 400) further comprises:
displaying the set-grouped thumbnail images that show a grouping based on laterality and views in the graphical user interface (300; 400).

10. A medical image management system (600) comprising:
a network communication interface (604) to receive healthcare studies;
a memory (610) coupled to the network communication interface (604) to store received healthcare studies;
a display screen coupled to the memory to display the received healthcare studies; and
one or more processors (602) coupled to the network communication interface (604), the memory (610), and the display screen and configured to:
receive an image data set from a data source, the image data set including projection X-ray mammographic images for a healthcare study;
determine an ungrouped thumbnail image for each projection X-ray mammographic image of the image data set, the ungrouped thumbnail images for display in a graphical user interface of a display device;
group images of the image data set into a plurality of sets;
determine a set-grouped thumbnail image for each set of grouped images, the set-grouped thumbnail images for display in the graphical user interface (300; 400);
generate a graphical user interface (300; 400) that includes displaying at least one of the set-grouped thumbnail images or the ungrouped thumbnail images in the graphical user interface (300; 400);
receive user input on a user interface element; and,
in response to receiving user input on the user interface element:
switch the graphical user interface (300; 400) from displaying set-grouped thumbnail images in the graphical user interface (300; 400) to displaying ungrouped thumbnail images in the graphical user interface (300; 400); or
switch the graphical user interface (300; 400) from displaying ungrouped thumbnail images in the graphical user interface (300; 400) to displaying set-grouped thumbnail images in the graphical user interface (300; 400).

11. The medical image management system (600) of claim 10, wherein the one or more processors (602) are further configured to:
select projection X-ray mammographic images from the image data set for display in the graphical user interface (300; 400); and
display the selected projection X-ray mammographic images in the graphical user interface (300; 400).

12. The medical image management system (600) of claim 10 or 11, wherein the one or more processors (602) configured to generate a graphical user interface (300; 400) that includes displaying at least one of the set-grouped thumbnail images or the ungrouped thumbnail images in the graphical user interface (300; 400) are further configured to:
generate a thumbnail bar element (330; 430), the thumbnail bar element (330; 430) configured to display at least one of the ungrouped thumbnail images or the set-grouped thumbnail images.

13. The medical image management system (600) of claim 12, wherein the one or more processors (602) configured to switch the graphical user interface (300; 400) from displaying set-grouped thumbnail images in the graphical user interface (300; 400) to displaying ungrouped thumbnail images in the graphical user interface (300; 400) are further configured to:
modify the generated graphical user interface (300; 400) to display the ungrouped thumbnail images in the thumbnail bar element (330; 430) in the graphical user interface (300; 400); and
display at least one of the ungrouped thumbnail images in the thumbnail bar element (330; 430).

14. The medical image management system (600) of claim 12 or 13, wherein the one or more processors (602) configured to switch the graphical user interface (300; 400) from displaying ungrouped thumbnail images in the graphical user interface (300; 400) to displaying set-grouped thumbnail images in the graphical user interface (300; 400) are further configured to:
modify the generated graphical user interface (300; 400) to display the set-grouped thumbnail images in the thumbnail bar element (330; 430) in the graphical user interface (300; 400); and
display at least one of the set-grouped thumbnail images in the thumbnail bar element (330; 430).

15. A non-transitory computer-readable storage media having instructions stored thereupon that when executed by a system (600) having at least one processor (602), a memory (610), and a display screen therein, cause the system to perform the method of any of claims 1 to 9.
